# EUROPÄISCHE PATENTSCHRIFT

(11) **EP 2 711 348 B1**
(45) Veröffentlichungstag und Bekanntmachung des Hinweises auf die Patenterteilung: **01.07.2015**
(21) Anmeldenummer: 12008169.0
(22) Anmeldetag: 06.12.2012
(51) Int. Cl.: C07C 2/84, C07C 4/04, C07C 5/327, C07C 11/04, C01B 3/38

(54) **Anlage und Verfahren zur Herstellung von Ethylen**
Method and device for producing ethylene
Installation et procédé destinés à la fabrication d'éthylène

(30) Priorität: 20.09.2012 DE 102012018602
(43) Veröffentlichungstag der Anmeldung: 26.03.2014
(73) Patentinhaber: Linde Aktiengesellschaft, 80331 München (DE)
(72) Erfinder: Göke, Volker, 82538 Geretsried (DE); Schmigalle, Holger, 82538 Geretsried (DE); Thaller, Christian, 80687 München (DE)

(56) Entgegenhaltungen:
- WO-A2-2007/045364
- DE-A1- 1 543 156
- DE-A1- 3 237 079
- DE-A1- 4 333 372
- NL-A- 9 300 168

## Beschreibung

Die Erfindung betrifft eine Anlage zur Erzeugung von Ethylen, insbesondere durch oxidative Kopplung von Methan, gemäß dem Oberbegriff des Anspruchs 1. Des Weiteren betrifft die Erfindung ein Verfahren zur Erzeugung von Ethylen.

Eine derartige Anlage weist einen Reaktor auf, in den zur oxidativen Kopplung von Methan Sauerstoff und Methan eingeleitet werden. Bei der Reaktion werden zunächst Methylradikale bei hohen Temperaturen (ca. 600-880°C) an einer katalytischen Oberfläche (z.B. zwei- oder mehrkomponentige Metalloxidkatalysator mit Alkali-, Erdalkalielementen und/oder Elementen aus der Gruppe der seltenen Erden) im Reaktor gebildet, die sodann in der Gasphase zu Ethan rekombinieren, das im weiteren Verlauf zu Ethylen umgewandelt wird, so dass im Ergebnis ein erster Stoffstrom enthaltend zumindest Ethan und Ethylen vorliegt. Die Anlage weist des Weiteren eine mit dem Reaktor strömungsmittelleitend verbundene Aufarbeitungseinheit auf, die zum Trennen des ersten Stoffstromes in zumindest einen C₁₋-Stoffstrom, der Kohlenwasserstoffe mit einem Kohlenstoffatom und ferner CO und H₂ aufweist, sowie in einen Ethylenproduktstrom ausgebildet ist, sowie eine mit der Aufarbeitungseinheit verbundene Trenneinrichtung (z.B. Druckwechseladsorptionseinrichtung), die dazu ausgebildet ist, den C₁₋- Stoffstrom in einen wasserstoffreichen Wasserstoffproduktstrom sowie einen wasserstoffarmen Restgasstrom zu trennen, der üblicherweise verfeuert wird.

Des Weiteren ist zur Ethylen- bzw. Olefinherstellung dje Dampfspaltung (Steamcracking) bekannt. Hierbei wird ein kohlenwasserstoffhaltiger Einsatz mit Wasserdampf gemischt, wobei das so gebildete Einsatzgas zur Spaltung üblicherweise durch metallische Rohre eines Spaltofens geführt wird, die zur Deckung des Wärmebedarfs des endothermen Spaltprozesses von außen mit Brennern beheizt werden. Der so erhaltene olefin- und wasserstoffhaltige Rohgasstrom wird üblicherweise gereinigt und in die gewünschten Olefine, insbesondere Ethylen, zerlegt.

NL 9300168 beschreibt ein Verfahren zur Umsetzung von Methan.

Der vorliegenden Erfindung liegt die Aufgabe zugrunde, eine Anlage bzw. ein Verfahren der eingangs genannten Art weiter zu verbessern.

Dieses Problem wird durch eine Anlage mit den Merkmalen des Anspruchs 1 gelöst.

Danach ist eine mit der Aufarbeitungseinheit verbundene Dampfspaltungseinrichtung zur Erzeugung eines olefin- und wasserstoffhaltigen Rohgasstromes vorgesehen, wobei die Aufarbeitungseinheit dazu ausgebildet ist, den Rohgasstrom zusammen mit dem ersten Stoffstrom in zumindest den C₁₋-Stoffstrom sowie den Ethylenproduktstrom zu trennen, wobei die Anlage dazu ausgebildet ist, zumindest einen Teil des Restgasstromes als Einsatz in den OCM-Reaktor zurückzuführen. Vorzugsweise werden der erste Stoffstrom sowie der Rohgasstrom in der Aufarbeitungseinheit zusammengeführt, wobei die einzelnen Ströme vor dem Zusammenführen vorgereinigt werden können. Aus dem zusammengeführten Strom wird sodann bevorzugt der C₁₋-Stoffstrom sowie der Ethylenproduktstrom abgetrennt.

Die erfindungsgemäße Verzahnung eines OCM-Reaktors mit einer Dampfspaltungseinrichtung (Streamcracker) ist von Vorteil, da der bei der Dampfspaltung erzeugte und in den OCM-Reaktor zurückgeführte Wasserstoff einerseits im OCM-Reaktor durch Wasserstoffverbrennung Energie bereitstellt und andererseits den Katalysator im Reaktor aktiviert sowie eine Verkokung im Reaktor vermindert .

Die Dampfspaltungseinrichtung, die zur Dampfspaltung einen oder mehrere Spaltöfen aufweisen kann, ist bevorzugt dazu ausgebildet, als Einsatz Kohlenwasserstoffe wie z.B. Ethan, Propan, Erdgaskondensate und/oder schwerere Einsätze, wie z.B. Naphtha, in Gegenwart von Wasserdampf unter Entstehung des insbesondere wasserstoff- und ethylenhaltigen Rohgases zu spalten.

Weiterhin ist die Aufarbeitungseinheit vorzugsweise dazu eingerichtet und vorgesehen, aus dem bei der oxidativen Kopplung von Methan erzeugten ersten Stoffstrom sowie dem bei der Dampfspaltung erzeugten Rohgasstrom einen ethan- und propanreichen Stoffstrom abzutrennen, der als Einsatz in die mit der Aufarbeitungseinheit entsprechend strömungsmittelleitend verbundene Dampfspaltungseinrichtung eingespeist wird.

Ferner ist bevorzugt auch die Trenneinheit strömungsmittelleitend mit der Dampfspaltungseinrichtung verbunden, wobei die Anlage dazu ausgebildet ist, zumindest einen Teil des Restgasstromes in die Dampfspaltungseinrichtung einzuspeisen, die dazu ausgebildet ist, jenen Teil des Restgasstromes zu unterfeuern, um die für die Dampfspaltung des vorgenannten Einsatzes (z.B. Ethan und/oder schwerere Einsätze wie Naphtha) benötigte Wärme bereitzustellen bzw. zu dieser beizutragen.

Des Weiteren kann die erfindungsgemäße Anlage dazu ausgebildet sein, einen Teilstrom des Wasserstoffproduktstromes in die Aufarbeitungseinheit zurückzuführen und dort insbesondere zum Hydrieren von Kohlenwasserstoffen zu verwenden.

Weiterhin wird das erfindungsgemäße Problem durch ein Verfahren zur Erzeugung von Ethylen mit den Merkmalen des Anspruchs 7 gelöst.

Danach wird bei dem erfindungsgemäßen Verfahren Sauerstoff und Methan in einen Reaktor eingeleitet, Methan im Reaktor unter Entstehung eines ersten Stoffstromes oxidativ gekoppelt, mittels Dampfspaltung eines kohlenwasserstoffhaltigen Einsatzes in einer Dampfspaltungseinrichtung ein olefin- und wasserstoffhaltiger Rohgasstrom erzeugt, der besagte erste Stoffstrom zusammen mit dem Rohgasstrom in einer Aufarbeitungseinheit in einen Ethylenproduktstrom und einen C₁₋-Stoffstrom aufgetrennt, der C₁₋-Stoffstrom in einer Trenneineit in einen Wasserstoffproduktstrom und einen wasserstoffarmen Restgasstrom getrennt, und zumindest ein Teilstrom des Restgasstromes als Einsatz in den Reaktor zurückgeführt.

Vorzugsweise wird weiterhin von dem besagten ersten Stoffstrom bzw. dem Rohgasstrom ein ethan- und propanreicher Stoffstrom abgetrennt, der als Einsatz in die Dampfspaltung gegeben wird.

Bevorzugt wird des Weiteren zumindest ein Teilstrom des Restgasstromes zur Erzeugung von Wärme für die besagte Dampfspaltung unterfeuert.

Weiterhin wird bevorzugt ein Teilstrom des Wasserstoffproduktstromes in die Aufarbeitungseinheit zurückgeführt, um dort z.B. zum Hydrieren von Kohlenwasserstoffen verwendet zu werden.

Weitere Einzelheiten und Vorteile der Erfindung sollen durch die nachfolgende Figurenbeschreibung eines Ausführungsbeispiels anhand der Figur erläutert werden.

Es zeigt:

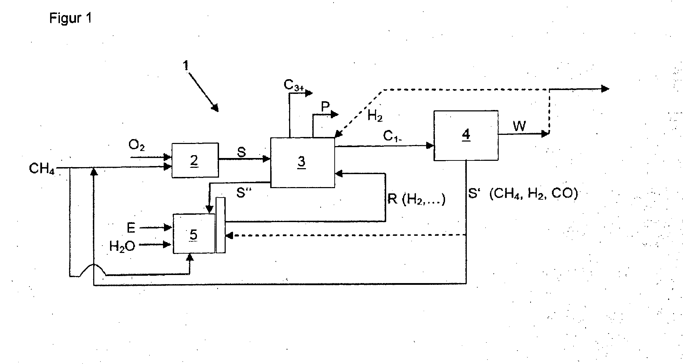

- Fig. 1: eine schematische Darstellung einer erfindungsgemäßen Anlage bzw. eines erfindungsgemäßen Verfahrens zur Erzeugung von Ethylen.

Figur 1 zeigt eine Anlage 1 zur Herstellung von Ethylen, die einen Reaktor 2 zur oxidativen Kopplung von Methan (sogenannter OCM-Reaktor), eine Aufarbeitungseinheit 3, eine Trenneinheit (z.B. Druckwechseladsorptionseinrichtung) 4 sowie eine Dampfspaltungseinrichtung 5 (Steamcracker) mit zumindest einem Spaltofen aufweist.

In den Reaktor 2 wird CH₄ und O₂ eingespeist, um im Reaktor 2 bei erhöhten Temperaturen Methan oxidativ an einer Katalysatoroberfläche zu koppeln. Hierbei entsteht ein erster Stoffstrom S, der u.a. Ethylen und Ethan enthält.

Des Weiteren weist die Anlage 1 eine Dampfspaltungseinrichtung 5 auf, in die als Einsatz E z.B. Ethan und/oder Naphtha sowie Wasserdampf eingespeist wird, wobei besagter Einsatz in der Dampfspaltungseinrichtung 5 unter Erzeugung eines Rohgasstromes R gespalten wird, der u.a. Olefine, insbesondere Ethylen, und Wasserstoff enthält.

In einer stromab des Reaktors 2 vorgesehen Aufarbeitungseinrichtung 3 wird der besagte erste ggf. vorgereinigte Stoffstrom S sowie der ggf. vorgereinigte Rohgasstrom R zusammengeführt und in einen C₃₊-Stoffstrom (Kohlenwasserstoffe mit drei oder mehr als drei Kohlenstoffatomen), einen Ethylenproduktstrom P, einen ethan- und propanreichen Stoffstrom S" sowie einen C₁₋-Stoffstrom aufgetrennt, wobei der C₁₋-Stoffstrom zur Erzeugung eines Wasserstoffproduktstromes W in die Trenneinheit 4 eingespeist wird.

Der in der Aufarbeitungseinrichtung 3 abgetrennte ethan- und propanreiche Stoffstrom S" wird als zusätzlicher Einsatz in die Dampfspaltungseinrichtung 5 gegeben.

In der Trenneinheit 3 wird der C₁₋-Stoffstrom in einen Wasserstoffproduktstrom W und einen wasserstoffarmen Restgasstrom S' getrennt. Hierzu kann z.B. eine Druckwechseladsorption verwendet werden, bei der der C₁₋-Stoffstrom bei erhöhtem Druck durch zumindest einen Adsorber gegeben wird, so dass Wasserstoff den mindestens einen Adsorber passiert und den besagten Wasserstoffproduktstrom W bildet, wobei die schwereren Komponenten, wie z.B. CH₄ und CO, an dem mindestens einen Adsorber adsorbiert werden und bei einem niedrigeren Druck desorbiert werden, woraus der wasserstoffarme Restgasstrom S' resultiert (dieser enthält insbesondere auch Wasserstoff aus einem Teilstrom des Wasserstoffproduktstromes W, der zum Spülen des mindestens einen Adsorbers verwendet wurde).

Der Restgasstrom S' wird sodann als Einsatz in den Reaktor 2 zurückgegeben. Des Weiteren kann ein Teil des Restgasstromes S' zum Unterfeuern in die Dampfspaltungseinrichtung 5 gegeben werden, um die für die Dampfspaltung benötigte Wärme zu generieren bzw. dazu beizutragen. Des Weiteren wird zum Unterfeuern vorzugsweise CH₄ verwendet, das stromauf des Reaktors 2 abgezweigt werden kann.

Schließlich kann ein Teil des Wasserstoffproduktstromes W zum Hydrieren von Bestandteilen des ersten Stoffstromes S bzw. des Rohgasstromes R verwendet werden (z.B. in der Aufarbeitungseinheit 3).

**Bezugszeichenliste**

| | |
|---|---|
| 1 | Anlage |
| 2 | OCM-Reaktor |
| 3 | Aufarbeitungseinheit |
| 4 | Trenneinheit (z.B. Druckwechseladsorptionseinrichtung) |
| 5 | Dampfspaltungseinrichtung |
| E | Einsatz für Dampfspaltung |
| S | Erster Stoffstrom |
| S' | Wasserstoffhaltiger Restgasstrom |
| S" | Ethan- und propanreicher Stoffstrom |
| R | Oefin- und wasserstoffhaltiger Rohgasstrom |
| P | Ethylenproduktstrom |
| W | Wasserstoffproduktstrom |

## Patentansprüche

1. Anlage zur Erzeugung von Ethylen, mit:
- einem Reaktor (2), der zur oxidativen Kopplung von Methan eingerichtet und vorgesehen ist,
- einer mit dem Reaktor (2) verbundenen Aufarbeitungseinheit (3), die zum Trennen eines bei der oxidativen Kopplung von Methan erzeugten ersten Stoffstromes (S) in zumindest einen C₁₋-Stoffstrom sowie einen Ethylenproduktstrom (P) ausgebildet ist, und
- einer mit der Aufarbeitungseinheit (3) verbundenen Trenneinheit (4), die dazu ausgebildet ist, den C₁₋- Stoffstrom zumindest in einen wasserstoffreichen Produktstrom (W) und einen wasserstoffarmen Restgasstrom (S') zu trennen,
**dadurch gekennzeichnet,**
**dass** eine mit der Aufarbeitungseinheit (3) verbundene Dampfspaltungseinrichtung (5) zur Erzeugung eines olefin- und wasserstoffhaltigen Rohgasstromes (R) vorgesehen ist, wobei die Aufarbeitungseinheit (3) dazu ausgebildet ist, den Rohgasstrom (R) zusammen mit dem ersten Stoffstrom (S) in zumindest den C₁₋-Stoffstrom sowie den Ethylenproduktstrom (P) zu trennen, wobei die Anlage (1) dazu ausgebildet ist, zumindest einen Teil des Restgasstromes (S') als Einsatz in den Reaktor (2) zurückzuführen.

2. Anlage nach Anspruch 1, **dadurch gekennzeichnet, dass** die Dampfspaltungseinrichtung (5) dazu ausgebildet ist, als Einsatz (E) einen oder mehrere der folgenden Stoffe in Gegenwart von Wasserdampf unter Entstehung des besagten Rohgasstromes (R) zu spalten: Kohlenwasserstoff, Ethan, Propan, Erdgaskondensate, und/oder Naphtha.

3. Anlage nach Anspruch 1 oder 2, **dadurch gekennzeichnet, dass** die Aufarbeitungseinheit (3) dazu ausgebildet ist, aus dem bei der oxidativen Kopplung von Methan erzeugten ersten Stoffstrom (S) und/oder dem Rohgasstrom (R) des Weiteren einen ethan- und propanreichen Stoffstrom (S") abzutrennen.

4. Anlage nach Anspruch 3, **dadurch gekennzeichnet, dass** die Aufarbeitungseinheit (3) derart mit der Dampfspaltungseinrichtung (5) verbunden ist, dass jener ethan- und propanreiche Stoffstrom (S") aus der Aufarbeitungseinheit (3) als Einsatz in die Dampfspaltungseinrichtung (5) einleitbar ist.

5. Anlage nach einem der vorhergehenden Ansprüche, **dadurch gekennzeichnet, dass** die Trenneinheit (4) derart mit der Dampfspaltungseinrichtung (5) verbunden ist, dass zumindest ein Teil des Restgasstromes (S') in die Dampfspaltungseinrichtung (5) einleitbar ist, wobei die Dampfspaltungseinrichtung (5) dazu ausgebildet ist, jenen Teil des Restgasstromes (S') zur Bereitstellung von Wärme in der Dampfspaltungseinrichtung (5) zu verfeuern.

6. Anlage nach einem der vorhergehenden Ansprüche, **dadurch gekennzeichnet, dass** die Trenneinheit (4) durch eine Druckwechseladsorptionseinheit gebildet wird, die dazu ausgebildet ist, den C₁₋- Stoffstrom mittels einer Druckwechseladsorption in den wasserstoffreichen Produktstrom (W) und den wasserstoffarmen Restgasstrom (S') zu trennen.

7. Verfahren zur Erzeugung von Ethylen, insbesondere unter Verwendung einer Anlage nach einem der vorhergehenden Ansprüche, bei dem
- Sauerstoff und Methan in einen Reaktor (2) eingeleitet werden,
- Methan im Reaktor (2) unter Entstehung eines ersten Stoffstromes (S) oxidativ gekoppelt wird,
- mittels Dampfspaltung eines kohlenwasserstoffhaltigen Einsatzes (E) in einer Dampfspaltungseinrichtung (5) ein olefin- und wasserstoffhaltiger Rohgasstrom (R) erzeugt wird,
- der besagte erste Stoffstrom (S) zusammen mit dem Rohgasstrom (R) in einer Aufarbeitungseinheit (3) in einen Ethylenproduktstrom (P) und einen C₁₋-Stoffstrom aufgetrennt wird,
- der C₁₋-Stoffstrom in einer Trenneineit (4) in einen Wasserstoffproduktstrom (W) und einen wasserstoffarmen Restgasstrom (S') getrennt wird, und
- zumindest ein Teilstrom des Restgasstromes (S') als Einsatz in den Reaktor (2) zurückgeführt wird.

8. Verfahren nach Anspruch 7, **dadurch gekennzeichnet, dass** von dem besagten ersten Stoffstrom (S) und/oder dem Rohgasstrom (R) in der Aufarbeitungseinheit (3) des Weiteren ein ethan- und propanreicher Stoffstrom (S") abgetrennt wird, der als Einsatz in die Dampfspaltungseinrichtung (5) zurückgeführt wird.

9. Verfahren nach Anspruch 7 oder 8, **dadurch gekennzeichnet, dass** zumindest ein Teilstrom des Restgasstromes (S') zur Erzeugung von Wärme für die Dampfspaltung in der Dampfspaltungseinrichtung (5) verfeuert wird.

10. Verfahren nach einem der Ansprüche 7 bis 9, **dadurch gekennzeichnet, dass** der C₁₋-Stoffstrom in der Trenneinheit (4) mittels einer Druckwechseladsorption in den Wasserstoffproduktstrom (W) und den wasserstoffarmen Restgasstrom (S') getrennt wird.

11. Verfahren nach einem der Ansprüche 7 bis 10, **dadurch gekennzeichnet, dass** der Einsatz (E) für die Dampfspaltung durch einen oder mehrere der folgenden Stoffe gebildet wird: Ethan, Propan, Erdgaskondensate, und/oder Naphtha.

## Claims

1. Plant for producing ethylene and comprising
- a reactor (2) set up and provided to effect oxidative coupling of methane,
- a work-up unit (3) connected to the reactor (2) and configured to separate a first material stream (S) produced during oxidative coupling of methane into at least a C₁₋ material stream and an ethylene product stream (P) and
- a separation unit (4) connected to the work-up unit (3) and configured to separate the C₁₋ material stream at least into a hydrogen-rich product stream (H) and a hydrogen-lean residual gas stream (S'),
**characterized in that**
the plant is provided with a steam cracker (5) for producing an olefin- and hydrogen-containing crude gas stream (C) and connected to the work-up unit (3), wherein the work-up unit (3) is configured to separate the crude gas stream (C) along with the first material stream (S) into at least the C₁₋ material stream and the ethylene product stream (P), wherein the plant (1) is configured to recycle at least a portion of the residual gas stream (S') into the reactor (2) as input.

2. Plant according to Claim 1, **characterized in that** the steam cracker (5) is configured to crack in the presence of steam an input (I) consisting of one or more substances selected from hydrocarbon, ethane, propane, natural gas condensates and naphtha to form said crude gas stream (C).

3. Plant according to either of Claims 1 and 2, **characterized in that** the work-up unit (3) is configured to additionally remove from the first material stream (S) produced during oxidative coupling of methane and/or from the crude gas stream (C) an ethane- and propane-rich material stream (S").

4. Plant according to Claim 3, **characterized in that** the work-up unit (3) is connected to the steam cracker (5) such that said ethane- and propane-rich material stream (S") from the work-up unit (3) is introducible into the steam cracker (5) as input.

5. Plant according to any preceding claim, **characterized in that** the separation unit (4) is connected to the steam cracker (5) such that at least a portion of the residual gas stream (S') is introducible into the steam cracker (5), wherein the steam cracker (5) is configured to fire said portion of the residual gas stream (S') in the steam cracker (5) to provide heat.

6. Plant according to any preceding claim, **characterized in that** the separation unit (4) is a pressure swing adsorption unit configured to separate the C₁₋ material stream into the hydrogen-rich product stream (H) and the hydrogen-lean residual gas stream (S') by pressure swing adsorption.

7. Process for producing ethylene, in particular using a plant according to any preceding claim, comprising
- introducing oxygen and methane into a reactor (2),
- effecting oxidative coupling of methane in the reactor (2) to form a first material stream (S),
- steam cracking a hydrocarbon-containing input (I) in a steam cracker (5) to produce an olefin- and hydrogen-containing crude gas stream (C),
- separating said first material stream (S) along with the crude gas stream (C) into an ethylene product stream (P) and a C₁₋ material stream in a work-up unit (3),
- separating the C₁₋ material stream into a hydrogen product stream (H) and a hydrogen-lean residual gas stream (S') in a separation unit (4) and
- recycling at least a substream of the residual gas stream (S') into the reactor (2) as input.

8. Process according to Claim 7, **characterized in that** it comprises further removing from said first material stream (S) and/or the crude gas stream (C) in the work-up unit (3) an ethane- and propane-rich material stream (S") which is recycled into the steam cracker (5) as input.

9. Process according to either of Claims 7 and 8, **characterized in that** at least a substream of the residual gas stream (S') is fired in the steam cracker (5) to produce heat for steam cracking.

10. Process according to any of Claims 7 to 9, **characterized in that** the C₁₋ material stream is separated into the hydrogen product stream (H) and the hydrogen-lean residual gas stream (S') by pressure swing adsorption in the separation unit (4).

11. Process according to any of Claims 7 to 10, **characterized in that** the input (I) for the steam cracking consists of one or more substances selected from ethane, propane, natural gas condensates and/or naphtha.

## Revendications

1. Installation destinée à la fabrication d'éthylène, comprenant:
- un réacteur (2), qui est conçu et prévu pour le couplage oxydant du méthane,
- une unité de retraitement (3) reliée au réacteur (2), qui est configurée pour la séparation d'un premier flux de substances (S) produit lors du couplage oxydant du méthane en au moins un flux de substances C₁₋ ainsi qu'un flux de produit d'éthylène (P), et
- une unité de séparation (4) reliée à l'unité de retraitement (3), qui est configurée pour séparer le flux de substances C₁₋ au moins en un flux de produit riche en hydrogène (W) et un flux de gaz résiduel pauvre en hydrogène (S'),
**caractérisée en ce qu'**il est prévu un dispositif de craquage à la vapeur (5) relié à l'unité de retraitement (3) pour la production d'un flux de gaz brut (R) contenant des oléfines et de l'hydrogène, dans laquelle l'unité de retraitement (3) est configurée pour séparer le flux de gaz brut (R) en même temps que le premier flux de substances (S) en au moins le flux de substance C₁₋ ainsi que le flux de produit d'éthylène (P), dans laquelle l'installation (1) est configurée pour renvoyer au moins une partie du flux de gaz résiduel (S') comme charge dans le réacteur (2).

2. Installation selon la revendication 1, **caractérisée en ce que** le dispositif de craquage à la vapeur (5) est configuré pour craquer comme charge (E) une ou plusieurs des substances suivantes en présence de vapeur d'eau avec formation dudit flux de gaz brut (R): hydrocarbure, éthane, propane, condensat de gaz naturel, et/ou naphte.

3. Installation selon la revendication 1 ou 2, **caractérisée en ce que** l'unité de retraitement (3) est configurée pour séparer en outre un flux de substance (S") riche en éthane et en propane à partir du premier flux de substance (S) produit lors du couplage oxydant de méthane et/ou à partir du flux de gaz brut (R).

4. Installation selon la revendication 3, **caractérisée en ce que** l'unité de retraitement (3) est reliée au dispositif de craquage à la vapeur (5) de telle manière que ce flux de substance riche en éthane et en propane (S") issu de l'unité de retraitement (3) puisse être introduit comme charge dans le dispositif de craquage à la vapeur (5).

5. Installation selon l'une quelconque des revendications précédentes, **caractérisée en ce que** l'unité de séparation (4) est reliée au dispositif de craquage à la vapeur (5), d'une manière telle qu'au moins une partie du flux de gaz résiduel (S') puisse être introduite dans le dispositif de craquage à la vapeur (5), dans laquelle le dispositif de craquage à la vapeur (5) est configuré pour brûler cette partie du flux de gaz résiduel (S') pour la production de chaleur dans le dispositif de craquage à la vapeur (5).

6. Installation selon l'une quelconque des revendications précédentes, **caractérisée en ce que** l'unité de séparation (4) est formée par une unité d'adsorption à pression alternée, qui est configurée pour séparer le flux de substance C₁₋ au moyen d'une adsorption à pression alternée en ledit flux de produit riche en hydrogène (W) et ledit flux de gaz résiduel pauvre en hydrogène (S').

7. Procédé destiné à la fabrication d'éthylène, en particulier en utilisant une installation selon l'une quelconque des revendications précédentes, dans lequel
- on introduit de l'oxygène et du méthane dans un réacteur (2),
- on effectue le couplage oxydant du méthane dans le réacteur (2) avec formation d'un premier flux de substance (S),
- par craquage à la vapeur d'une charge (E) contenant des hydrocarbures dans un dispositif de craquage à la vapeur (5), on produit un flux de gaz brut (R) contenant des oléfines et de l'hydrogène,
- on sépare ledit premier flux de substance (S) en même temps que le flux de gaz brut (R) dans une unité de retraitement (3) en un flux de produit d'éthylène (P) et un flux de substance C₁₋,
- on sépare le flux de substance C₁₋ dans une unité de séparation (4) en un flux de produit d'hydrogène (W) et un flux de gaz résiduel pauvre en hydrogène (S'), et
- on renvoie au moins un flux partiel du flux de gaz résiduel (S') comme charge dans le réacteur (2).

8. Procédé selon la revendication 7, **caractérisé en ce que** l'on sépare en outre dudit premier flux de substance (S) et/ou du flux de gaz brut (R) dans l'unité de retraitement (3) un flux de substance riche en éthane et en propane (S"), que l'on renvoie comme charge dans le dispositif de craquage à la vapeur (5).

9. Procédé selon la revendication 7 ou 8, **caractérisé en ce que** l'on brûle au moins un flux partiel du flux de gaz résiduel (S') pour produire de la chaleur pour le craquage à la vapeur dans le dispositif de craquage à la vapeur (5).

10. Procédé selon l'une quelconque des revendications 7 à 9, **caractérisé en ce que** l'on sépare le flux de substance C₁₋ dans l'unité de séparation (4) au moyen d'une adsorption à pression alternée en ledit flux de produit d'hydrogène (W) et ledit flux de gaz résiduel pauvre en hydrogène (S').

11. Procédé selon l'une quelconque des revendications 7 à 10, **caractérisé en ce que** l'on forme la charge (E) pour le craquage à la vapeur par une ou plusieurs des substances suivantes: éthane, propane, condensat de gaz naturel, et/ou naphte.
